# EUROPEAN PATENT APPLICATION

(11) **EP 2 524 674 A1**
(43) Date of publication of application: **21.11.2012**
(21) Application number: 12168216.5
(22) Date of filing: 16.05.2012
(51) Int. Cl.: A61F 5/01

(54) **Lower leg-foot orthosis**

(30) Priority: 16.05.2011 NL 2006788
(71) Applicant: Buchrnhornen B.V., 5623 BB Eindhoven (NL)
(72) Inventor: Buchrnhornen, Petrus Cornelis, 5623 BB Eindhoven (NL)
(74) Representative: Eveleens Maarse, Pieter

(57) **Abstract**

The invention relates to a lower leg-foot orthosis for controlling the relative position of the foot and the lower leg of a person wearing the orthosis, wherein the orthosis is provided with a foot section for placing under a foot, the longitudinal direction of which corresponds to the longitudinal direction of the foot, and a lower leg section for placing against the lower leg, the longitudinal direction of which corresponds to the longitudinal direction of the lower leg, and a bendable heel section which connects the foot section to the lower leg section and the bending direction of which lies substantially in a plane in which the longitudinal direction of the foot and of the lower leg is located, wherein the heel section extends a certain distance between the foot section and the lower leg section, and the bending of the heel section is distributed uniformly over this distance. Enlarging the bending area makes the degree of bending smaller so that fatigue is reduced and the lifespan of the orthosis is increased.

## Description

The invention relates to a lower leg-foot orthosis for controlling the relative position of the foot and the lower leg of a person wearing the orthosis. Such orthoses are worn by people wherein owing to a disorder the muscles determining the position of the foot relative to the lower leg are no longer effective, be it as a result of a disorder of these muscles or as a result of a neurological condition, usually a CVA. So-called `foot drop' is one of the types of disorder for which such an orthosis is used. The resilient action of the orthosis takes on the muscle function. The orthosis further provides stability of the horizontal rotation position of the foot relative to the lower leg.

The object of such an orthosis is to determine the position of the foot relative to the lower leg of the user, particularly the angle between the longitudinal direction of the foot and the longitudinal direction of the lower leg. The orthosis is after all applied for people wherein this functionality has been lost. Known for the purpose of achieving this function control is an orthosis comprising:
- a foot section for placing under a foot of the wearer and extending substantially horizontally in the rest position, the longitudinal direction of which corresponds to the longitudinal direction of the foot;
- a lower leg section for placing against the rear side of the lower leg of the leg of the wearer associated with the relevant foot and extending substantially vertically in the rest position, the longitudinal direction of which corresponds to the longitudinal direction of the lower leg; and
- a heel section connecting the foot section to the lower leg section and being resiliently bendable when a relevant force is exerted, the bending direction of which lies substantially in a plane in which the longitudinal direction of the foot and of the lower leg of the person wearing the orthosis is located.

The flexibility of the heel section substantially determines the behaviour of the orthosis here, in particular the position of the foot relative to the position of the lower leg. This flexibility is usually chosen such that in the rest position the angle between the longitudinal direction of the foot and the longitudinal direction of the lower leg is about 90°. Starting from this position, the position of the foot can bend up to about 40° upward and, when the foot is stretched, bend about 7° downward. The orthosis makes it possible in many cases for the wearer to walk. When the wearer walks with the orthosis, the heel section is bent at each step. In many orthoses this results in rapid wear of the heel section, so that the flexibility of the heel section changes and this flexibility no longer has the desired value, so that an unstable gait results and the orthosis no longer fulfills its function properly. Such an orthosis has to be replaced.

The object of the invention is to provide an orthosis with a longer lifespan which enables a stable gait.

This object is achieved with an orthosis of the above stated type, wherein the heel section extends a certain distance between the foot section and the lower leg section, and the bending of the heel section is distributed uniformly over this distance.

Research has shown that the decrease in resilience is caused by phenomena similar to material fatigue. These are caused by the concentration of the bending in a small area. This area is thereby bent sharply and the material in question is heavily loaded. Enlarging the area where bending occurs makes the rate of bending per area smaller so that the load greatly decreases, there is a reduction in the fatigue phenomena and the lifespan of the orthosis is increased, as tests have demonstrated.

Bending occurs in the already bent heel section. Each part of this heel section thus has a certain radius of curvature in unloaded state. When the heel section bends, this radius of curvature decreases and measures according to the invention ensure a relatively uniform decrease in the radius of curvature during bending. This greatly reduces the change in the radius of curvature and thereby the deformation of the heel section. In other words, the flexibility of all the parts of the heel section is the same so that the radius of curvature decreases by the same percentage everywhere. It is noted that it is attractive for the bending stiffness to increase in the case of a sharp bend, i.e. shortly before the maximum bending angle is reached. This is however related more to the functionality of the orthosis, i.e. support of the foot, than to the lifespan.

Because the forces acting on the heel section and the material fatigue caused thereby depend not only on the relative change in the radius of curvature but also on the radius of curvature itself, it is attractive for the heel section in the unloaded state to have a constant radius of curvature. In the unloaded situation the curvature is then already distributed along the length of the heel section so that the radius of curvature has as far as possible the same value everywhere. The minimum value of the radius of curvature is hereby as great as possible. It hereby also becomes easier to distribute as constantly as possible the relative change in the radius of curvature occurring during bending.

According to another preferred embodiment, the bending of the heel section is represented by a virtual rotation point and the orthosis is dimensioned for the purpose of positioning the virtual rotation point at the position of the natural rotation point between the lower leg and the foot after being placed on a wearer. This measure results in a great increase in wearer comfort; this is because the rotation points now coincide as far as possible so that respectively the foot and foot section and the lower leg and lower leg section connected to the rotation points move as little as possible relative to each other during rotation, and the friction which is associated with the relative movement and which decreases wearer comfort is minimized.

As alternative to the above elucidated embodiment, the orthosis can be dimensioned for the purpose of positioning the virtual rotation point at a position behind and under the natural rotation point between the lower leg and the foot after being placed on a wearer. This prevents pressure occurring on the rear side of the heel when the orthosis is bent forward. In this embodiment the heel section therefore usually has a smaller radius of curvature.

As set forth above, the bending must take place in the heel section. This necessitates the heel section being less stiff than the lower leg section or the foot section. It is then structurally attractive for the average width of the heel section to be smaller than the average width of the foot section or of the lower leg section. This also has the advantage that the small width of the heel section makes it possible for the orthosis to be worn in a shoe, sometimes even an off-the-shelf shoe. This reduces the need for orthopaedic shoes, which reduces costs and makes it possible to serve a wider market segment.

According to a preferred embodiment, the heel section has an at least partially concave form toward the inside. The heel section hereby fits better on the heel and so fits better in the shoe. This also results in a greater stiffness of the heel section, which usually provides the option of giving the heel section a narrower form.

The above stated measures require a certain degree of stiffness of the orthosis, combined with a sufficiently small thickness. These requirements can be met when the lower leg section, the heel section and the foot section each have a flat, possibly arched structure and are formed substantially from a single piece of fibre composite. The single piece of fibre composite further enables an easy method of manufacture, whereby fitting to the wearer can take place in simple manner. It hereby becomes possible to make large numbers of semi-manufactures which can be easily fitted to the users, which results in a considerable cost saving. It is noted that a single fibre composite can comprise multiple layers. This also avoids the decrease in resilience which can be largely attributed to phenomena in thermoplastics similar to fatigue. A longer lifespan is possible as a result. A final advantage is a smaller thickness, so that the orthosis can take a thinner form and can be worn in an off-the-shelf shoe.

The required strength and stiffness can best be achieved when the majority of the fibres of the fibre composite extends with a directional component in the longitudinal direction of the orthosis. The fibres can be arranged as different layers, although it is also possible for the fibres to be arranged in rovings with mutually differing direction.

During use the orthosis is not only loaded by forces in the longitudinal direction (in the direction of the sagittal axis) but also by torsional forces. For the purpose of absorbing these forces a preferred embodiment provides the measure that the fibre composite comprises a first, a second and a third group of fibres, the first group of fibres extends substantially in the longitudinal direction of the orthosis, the second group of fibres extends at an acute first angle relative to the first group of fibres and that the third group of fibres extends at a second angle, differing from the first angle, relative to the first group of fibres. The fibres belonging to the different groups can be arranged in different layers, although it is also possible for the groups of fibres to be arranged in rovings with mutually differing directions.

The most heavily loaded parts of the orthosis are situated in the heel section and in the parts of the foot section and the lower leg section connecting directly to the heel section. The fibres in the heel section are under strain of tension during bending of the heel section, particularly the fibres located on the outer side of the heel section. This is likewise the case for the fibre pieces connecting thereto in the foot section and the calf section because the fibres transmit the tensile forces, to the extent they are not absorbed by the matrix of the fibre composite, to the fibre pieces in these adjoining parts of the orthosis. In order to give these parts of the foot section and the heel section a sufficiently stiff form it is recommended that the fibre composite comprises at least two layers of fibres, that at least one of the layers of fibres extends over only a part of the length of the orthosis and that the number of layers of fibres in the heel section is greater than the number of layers at the distal ends of the orthosis.

In order to increase wearer comfort, it is recommended that the inner side of the heel section is covered with a covering layer of a material which is softer than the fibre composite. Such a material is formed for instance by rubber or a plastic with rubber-like properties.

The outer side of the heel section can also be covered with a covering layer of a material which is softer than the fibre composite. This usually has a decorative effect.

In order to improve the adhesion between the soft layer or layers and the material of the actual orthosis, according to a preferred embodiment the covering layer is arranged prior to curing of the fibre composite and the covering layer is cured together with the fibre composite, so that the material of the covering layer extends into the fibre composite. This measure also has the effect that the soft material extends to some extent into the matrix of the composite so that this matrix material is also softer.

In order to improve the coupling between the lower leg and the lower leg section, the lower leg section is provided with a flat, possibly arched structure which partially coincides with the part of the lower leg section formed by the fibre composite and which extends to a position outside this part. This structure is preferably bent so that it extends partially around the lower leg. Possibly occurring spasms are hereby also prevented.

For the purpose of obtaining a better fixation between foot and shoe, the foot section is preferably provided with a flat, possibly arched structure partially which coincides with the part of the foot section formed by the fibre composite and which extends to a position outside this part.

The structure partially coinciding with the lower leg section or the foot section is preferably formed by a second fibre composite which is less stiff than the fibre composite from which the actual orthosis is made.

In order to obtain a good connection between the substantially flat structures and the actual orthosis, the fibre composite comprises at least two layers at the position of the foot section or the lower leg section and the structures extend partially between two of these layers.

The invention also relates to an orthosis adapted to be placed with its foot section into a shoe, wherein the foot section is covered by a shoe insert and a combination of such an orthosis, a shoe dimensioned for the orthosis and a shoe insert dimensioned for the shoe and the orthosis.

The invention will be elucidated hereinbelow with reference to the accompanying drawings, in which:
Figure 1 is a perspective schematic view of a lower leg-foot orthosis according to the invention;
Figure 2 is a schematic vertical cross-sectional view of a heel section of the orthosis according to the invention;
Figure 3 is a schematic horizontal cross-sectional view of the heel section shown in figure 2;
Figure 4 is a diagram of the fibres showing the direction of the fibres within the composite;
Figure 5 is a schematic vertical cross-sectional view of an orthosis according to the invention; and
Figure 6 is a schematic side view, partially in cross-sectional view, of a combination of a shoe and an orthosis according to the invention placed therein.

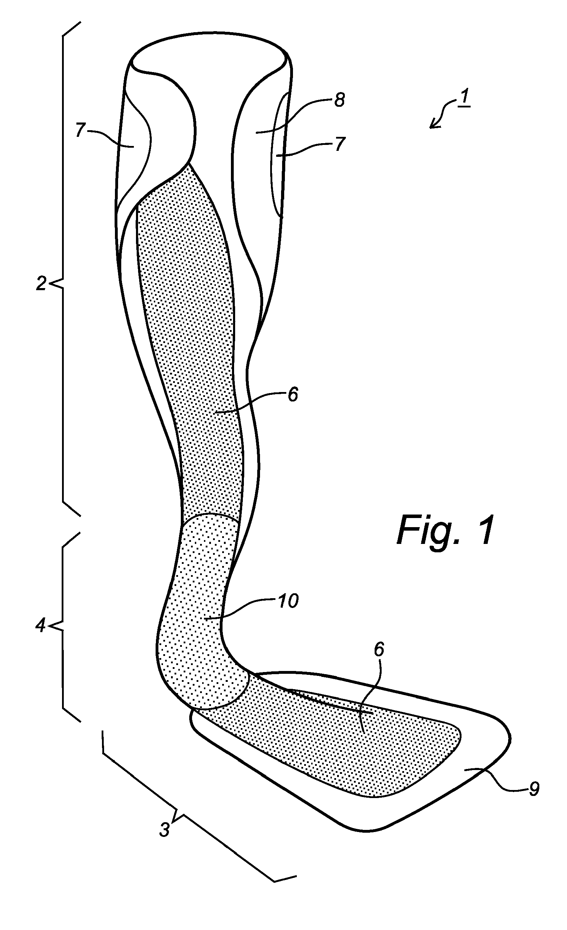

Figure 1 shows a lower leg-foot orthosis designated as a whole with 1. This orthosis 1 comprises a substantially vertically extending upper leg section 2, a substantially horizontally extending foot section 3 and a heel section 4 connecting the upper leg section 2 to the foot section. The whole orthosis 1 is manufactured from two-dimensional material. The lower leg section in particular is bent for the purpose of adjustment to the shape of the lower leg and the foot of the wearer of the orthosis.

Orthosis 1 is manufactured in large part from composite material, i.e. fibres embedded in a matrix of a synthetic resin, preferably a thermosetting synthetic resin. Use can for instance be made as fibres of metal fibres, carbon fibres, glass fibres or natural fibres. Other types of fibre are not precluded. The part of orthosis 1 manufactured from this fibre composite comprises at least a layer 6 on the inner side, shown hatched, and a layer 7 on the outer side, the contours of which are shown. The lower leg part 2 of the orthosis also comprises a part 8 which is manufactured from a softer but still shape-retaining thin material and which extends to a position outside the part 6, 7 manufactured from composite material. Part 8 is bent more sharply and serves to clamp the lower leg section fixedly onto the lower leg of the wearer. Foot section 3 also comprises such a part 9 extending to a position outside the part 6, 7 manufactured from composite material. At the position of heel section 4 a layer 10 of soft material is arranged on the inner side of the orthosis, while a layer 11 of soft material, which is however not shown in figure 1, is likewise arranged on the outer side of the heel section.

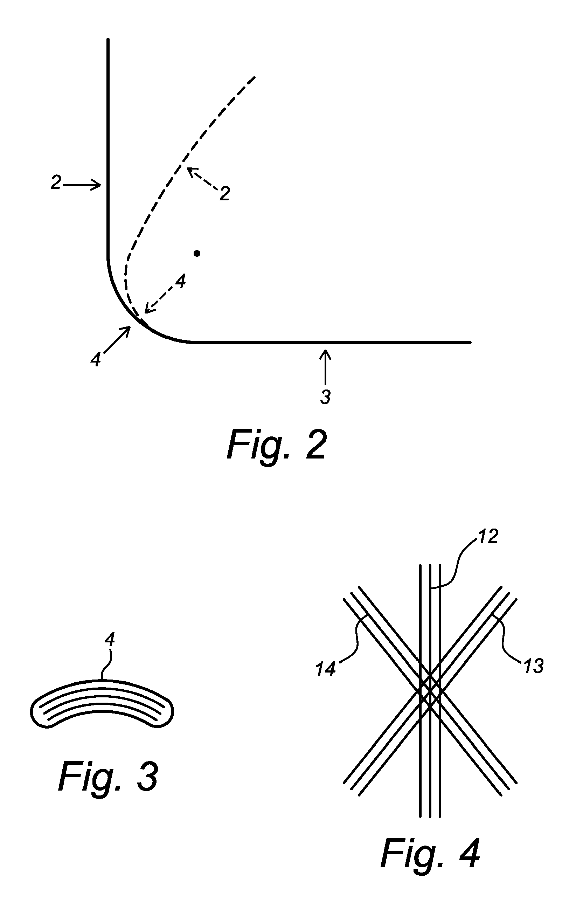

Figure 2 shows a vertical cross-section of the orthosis in schematic form. The position of the lower leg section 2, heel section 4 and foot section 3 is shown here in the unloaded, neutral form with a full line, and the position of these parts in the bent position is represented with a broken line. This figure shows in the first instance that the degree of bending, i.e. the ratio of the radius of curvature in the rest position and the radius of curvature in the bent position, is roughly the same. The degree of bending determines the wear, which in the case of a fibre composite material is defined largely by the degree of breakage of the usually non-stretchable fibres. Owing to the measure according to the invention the degree of bending does not become extreme anywhere, so the fibres do not break, or hardly so, and the lifespan is optimal. This figure also shows the virtual rotation point 35 of the orthosis. This virtual rotation point can coincide with the natural rotation point of the ankle, i.e. between the foot and the lower leg, although it is also possible for the virtual rotation point of the orthosis to lie below and behind (as seen in the direction of movement) the natural rotation point of the ankle. The natural rotation point of the ankle then lies above and in front of the virtual rotation point of the foot.

The figure further shows that the heel is formed substantially as a circular arc so that the radius of curvature is substantially the same everywhere. The stresses also already present in the rest position are hereby distributed well and thereby as low as possible. When the orthosis is bent because the user moves his/her foot upward, for instance during walking, the situation shown with a broken line results. This shows that the radius of curvature is still substantially the same so that the degree of bending, i.e. the change in the radius of curvature at rest to the radius of curvature in the bent position, is as equal as possible everywhere in order to prevent breaking of the fibres.

As already stated in the preamble, and as shown in figure 3, the heel section has an inward directed curve in the horizontal plane. This results in the sufficient stiffness in the upper part of the heel section, even in the case of a relatively narrow embodiment of the heel section.

Figure 4 shows how the fibres extend in the composite material. A first bundle of unidirectional fibres (usually referred to as a roving) 12 extends in the longitudinal direction of the orthosis. The fibre roving 12 extending in this direction imparts strength to the composite in the longitudinal direction. Also present is a second fibre roving 13 extending at an angle of about 30 °relative to first roving 12, while a third fibre roving 14 is shown extending at an angle of about -30° relative to first roving 12. It will be apparent that a fibre web as applied in the orthosis according to the invention comprises several of each of the fibre rovings 12, 13, 14. These rovings are assembled into a web in a weaving process. The fibre rovings 13, which extend at a different angle relative to fibre rovings 12, serve here to impart torsional stiffness to orthosis 1. It is noted that this configuration is in no way limitative; numerous other types of fibre configuration are possible.

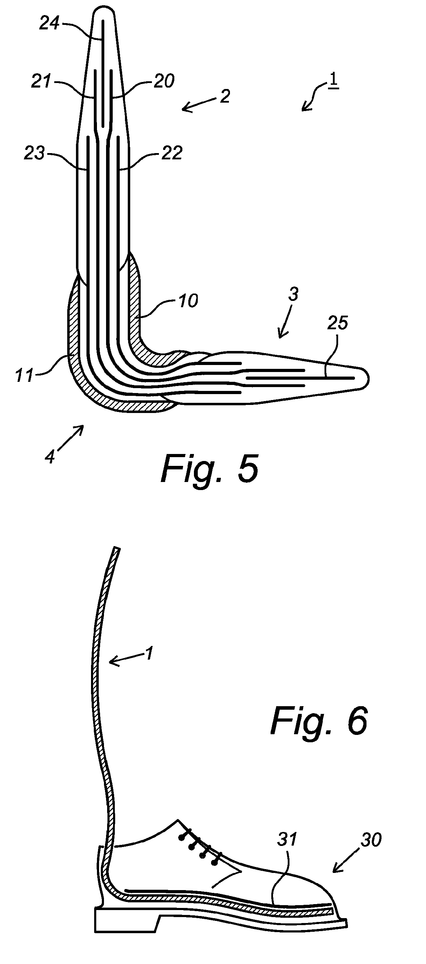

In contrast to figure 3, the cross-section of the orthosis shown in figure 5 shows the whole orthosis and also the assembly of the layers of the orthosis. In the exemplary embodiment shown in this figure the orthosis comprises the layer of fibre composite 6. This layer 6 comprises two layers of fibre web 20, 21 extending over the whole length of that part of the orthosis formed by the fibre composite material 6. This part further comprises two layers 22, 23 which extend over only a part of the length.

Lower leg section 2 is provided with a layer of textile composite 8 which is less stiff than the fibre composite material 6. This layer comprises a textile web 24 which, together with its matrix of synthetic resin, extends to a position outside the layer of fibre composite material 6. Foot section 3 is provided with a layer of textile composite 9 which is less stiff than the fibre composite material 6. This layer comprises a textile web 25 which, together with its matrix of synthetic resin, extends to a position outside the layer of fibre composite material 6. The matrix of the textile composite layers 24, 25 is formed from the same material as the matrix of the fibre composite material 6. The use of a different matrix material is however not precluded. It is also possible to use the matrix material without adding fibre material.

Arranged against the inner side of heel section 4 is a layer 10 of rubber-like material, and a layer 11 of the same material is arranged against the outer side of heel section 4. This increases the wearer comfort. The soft material also contributes toward damping of the bending of the heel section. As already stated above, the layers are arranged in liquid form prior to curing of the matrix of the composite materials. Vulcanization of the rubber takes place simultaneously with the curing. The use of a vulcanizable material goes together well with the use of a thermosetting matrix because both materials are cured by a heating process.

Finally, figure 6 shows a shoe 30 in which a foot section 3 and a heel section 4 of an orthosis 1 according to the invention are placed. The figure shows that the foot section is situated under a shoe insert 31 placed in shoe 30. Such shoe inserts 31 are already frequently applied in orthopaedics, and the use above the foot section results in an improved wearer comfort.

## Claims

1. Lower leg-foot orthosis for controlling the relative position of the foot and the lower leg of a person wearing the orthosis, wherein the orthosis comprises:
- a foot section adapted for placing under a foot of the wearer and extending substantially horizontally in the unloaded position, the longitudinal direction of which corresponds to the longitudinal direction of the foot;
- a lower leg section adapted for placing against the lower leg of the wearer associated with the relevant foot and extending substantially vertically in the unloaded position, the longitudinal direction of which corresponds to the longitudinal direction of the lower leg; and
- a heel section connecting the foot section to the lower leg section and being resiliently bendable when a relevant force is exerted, the bending direction of which lies substantially in a plane in which the longitudinal direction of the foot and of the lower leg of the person wearing the orthosis is located,
**characterized in that** the heel section extends a certain distance between the foot section and the lower leg section, and that the bending of the heel section is distributed uniformly over this distance.

2. Orthosis as claimed in claim 1, **characterized in that** the heel section in the unloaded state has a constant radius of curvature.

3. Orthosis as claimed in claim 1 or 2, **characterized in that** the bending of the heel section is represented by a virtual rotation point and that the orthosis is dimensioned for the purpose of positioning the virtual rotation point at the position of the natural rotation point between the lower leg and the foot after being placed on a wearer.

4. Orthosis as claimed in claim 1 or 2, **characterized in that** the bending of the heel section is represented by a virtual rotation point and that the orthosis is dimensioned for the purpose of positioning the virtual rotation point at a position behind and under the natural rotation point between the lower leg and the foot after being placed on a wearer.

5. Orthosis as claimed in claim 1, 2, 3 or 4, **characterized in that** the average width of the heel section is smaller than the average width of the foot section or of the lower leg section.

6. Orthosis as claimed in any of the foregoing claims, **characterized in that** the lower leg section and the foot section have a flat, possibly arched structure and that they are formed substantially from a single piece of fibre composite.

7. Orthosis as claimed in claim 6, **characterized in that** the majority of the fibres of the fibre composite extends with a component in the longitudinal direction of the orthosis.

8. Orthosis as claimed in claim 7, **characterized in that** the fibre composite comprises at least a first, a second and a third group of fibres, that the first group of fibres extends substantially in the longitudinal direction of the orthosis, that the second group of fibres extends substantially at an acute first angle relative to the first group of fibres and that the third group of fibres extends substantially at a second angle, differing from the first angle, relative to the first group of fibres.

9. Orthosis as claimed in claim 6, 7 or 8, **characterized in that** the fibre composite comprises at least two layers of fibres, that at least one of the layers of fibres extends over only a part of the length of the orthosis and that the number of layers of fibres in the heel section is greater than the number of layers at the distal ends of the orthosis.

10. Orthosis as claimed in claim 6, 7, 8 or 9, **characterized in that** the inner side of the heel section is covered with a covering layer of a material which is softer than the fibre composite.

11. Orthosis as claimed in any of the claims 6-10, **characterized in that** the outer side of the heel section is covered with a covering layer of a material which is softer than the fibre composite.

12. Orthosis as claimed in claim 10 or 11, **characterized in that** the covering layer is arranged prior to curing of the fibre composite and that the covering layer is cured together with the fibre composite, so that the material of the covering layer extends into the fibre composite.

13. Orthosis as claimed in any of the claims 6-12, **characterized in that** the lower leg section or the foot section is provided with a flat, possibly arched structure which partially coincides with the part of the lower leg section which is formed by the fibre composite and which extends to a position outside this part.

14. Orthosis as claimed in any of the foregoing claims, **characterized in that** the orthosis is adapted to be placed with its foot section into a shoe, wherein the foot section is covered by a shoe insert.

15. Combination of an orthosis as claimed in claim 14, a shoe dimensioned for the orthosis and a shoe insert dimensioned for the shoe and the orthosis.
